# EUROPEAN PATENT SPECIFICATION

(11) **EP 3 592 840 B1**
(45) Date of publication and mention of the grant of the patent: **31.05.2023**
(21) Application number: 18711247.9
(22) Date of filing: 02.03.2018
(51) Int. Cl.: C12N 5/00

(54) **CULTURE MEDIUM COMPRISING DIPEPTIDES**
KULTURMEDIUM MIT PEPTIDEN
MILIEU DE CULTURE COMPRENANT DES DIPEPTIDES

(30) Priority: 09.03.2017 DE 102017203908
(43) Date of publication of application: 15.01.2020
(73) Proprietor: Evonik Operations GmbH, 45128 Essen (DE)
(72) Inventor: MERZ, Friedhelm, 55283 Nierstein (DE); KARAU, Andreas, 63571 Gelnhausen (DE); HERMANN, Thomas, 63776 Mömbris (DE); KNAUP, Günter, 63486 Bruchköbel (DE)
(74) Representative: Evonik Patent Association
(86) International application number: PCT/EP2018/055197
(87) International publication number: WO 2018/162353

(56) References cited:
- WO-A1-2016/156476
- WO-A1-2016/196940
- MATTHEW R. HAYWARD ET AL: "Temperature and Oxygen Dependent Metabolite Utilization by Salmonella enterica Serovars Derby and Mbandaka", PLOS ONE, vol. 10, no. 3, 23 March 2015 (2015-03-23) , page e0120450, XP055469015, DOI: 10.1371/journal.pone.0120450
- PING XU ET AL: "Effects of glutamine and asparagine on recombinant antibody production using CHO-GS cell lines", BIOTECHNOLOGY PROGRESS, vol. 30, no. 6, 8 November 2014 (2014-11-08), pages 1457-1468, XP055190257, ISSN: 8756-7938, DOI: 10.1002/btpr.1957
- Andres Sánchez-kopper ET AL: "Tracking dipeptides atwork-uptake andintracellular fate inCHO culture", , 22 July 2016 (2016-07-22), page 48, XP055468583, DOI: 10.1186/s13568-016-0221-0 Retrieved from the Internet: URL:https://www.ncbi.nlm.nih.gov/pmc/artic les/PMC4958091/pdf/13568_2016_Article_221. pdf [retrieved on 2018-04-19]
- SWAN J M ET AL: "The synthesis of L-Glutaminyl-L-asparagine, L-GLUTAMINE AND L-ISOGLUTAMINE FROM P-TOLUENESULFONYL-L-GLUTAMIC ACID", JOURNAL OF THE AMERICAN CHEMICAL SOCIETY, AMERICAN CHEMICAL SOCIETY, vol. 76, 1 January 1954 (1954-01-01), pages 3110-3113, XP002269297, ISSN: 0002-7863, DOI: 10.1021/JA01641A002
- Li-Xiang Zhang ET AL: "Responses of CHO-DHFR cells to ratio of asparagine to glutamine in feed media: cell growth, antibody production, metabolic waste, glutamate, and energy metabolism", Bioresources and Bioprocessing, vol. 3, no. 1, 8 February 2016 (2016-02-08), XP055715739, DOI: 10.1186/s40643-015-0072-6
- Fatemeh Torkashvand ET AL: "Designed Amino Acid Feed in Improvement of Production and Quality Targets of a Therapeutic Monoclonal Antibody", PLOS ONE, vol. 10, no. 10, 19 October 2015 (2015-10-19), page e0140597, XP055704734, DOI: 10.1371/journal.pone.0140597
- KIM DO YUN ET AL: "Fed-batch CHO cell t-PA production and feed glutamine replacement to reduce ammonia production", BIOTECHNOLOGY PROGRESS, WILEY-BLACKWELL PUBLISHING, INC, US, vol. 29, no. 1, 1 January 2013 (2013-01-01), pages 165-175, XP009191145, ISSN: 1520-6033

## Description

### FIELD OF THE INVENTION

The present invention relates to biotechnological production processes. More specifically, the present invention relates to improved culture media for use in biotechnological production processes, processes employing such improved media, and to products obtained from the processes using the improved culture media.

### BACKGROUND OF THE INVENTION

Biotechnological processes are widely used for the production of biological products. These processes typically involve the cultivation of cells in a culture medium under conditions permissible to the growth and product formation by the cultivated cells. Cells useful in biotechnological production are bacterial cells, fungal cells, yeast cells, and cells of animal or plant origin.

Animal cell culture has long been used for the production of biological products, such as therapeutic proteins, polypeptides, oligopeptides or other biological molecules, such as therapeutic polysaccharides. In such cell culture processes, animal cells, normally genetically modified to produce a desired product, are cultivated in a liquid, solid or semi solid culture medium for cell proliferation and product formation. One significant advantage of cell culture is the fact that animal cells and plant cells are able to perform post-translational modifications of the primary product, such as folding and post-translational modification of a polypeptide.

In biotechnological production processes, in particular in animal cell cultures, control and optimization of the cell culture conditions is critical for cell proliferation and product formation. One decisive factor is medium composition. The concentration and quality of the final cell culture product depends heavily on the medium composition. Animal and plant cell cultures are particularly demanding in terms of the required nutrient composition and culture conditions. Required nutrients not only include basic sources for carbon, nitrogen and energy, such as sugar and ammonia, but also more complex nutrients, such as essential amino acids, and vitamins may be required. For this reason, supplementation of animal cell culture media with complex nutrient compositions, such as serum, has been used to provide the animal cells with a broad variety of nutrients. However, regulatory and safety concerns, as well as problems with the heterogeneity of the available sources of sera has led the industry to strive towards eliminating serum and other non-defined media from industrial cell culture processes. Cell cultures grown in serum-free media, however, often show nutritional deficiencies. For this reason, much effort has been given to identify those nutritional components of complex media, as well as their optimal concentrations, which are required for satisfactory growth and protein formation in cell cultures.

The supply of cell cultures with amino acids is known to have a significant effect on growth rate and production. Glutamine is routinely used in cell culture media, since it is an important source of carbon, nitrogen and energy for the cultured cells. It was demonstrated that the amount of glutamine necessary for optimal growth of animal cell cultures is 3 to 10 times greater than the amount of other amino acids (Eagle et al., Science 130:432-37). However, glutamine as a nutrient is unstable when dissolved in water at elevated temperatures, such as heat sterilizing conditions, because pyroglutamate and ammonia are formed under heat (Roth et al. 1988, In Vitro Cellular & Developmental Biology 24(7): 696-98). For this reason, glutamate is often used in cell culture media instead of glutamine (Cell Culture Technology for Pharmaceutical and Cell-based Therapies, 52, Sadettin et al., Eds., Taylor and Francis Group, 2006). Other groups have tried to avoid the formation of unwanted substances, such as pyroglutamate and ammonia, by adding glutamine-containing dipeptides, such as alanyl-glutamine or glycyl-glutamine, to the cell culture medium (Roth et al. (1988), In Vitro Cellular & Developmental Biology 24(7): 696-98). Yet other groups have proposed acylating dipeptides in order to make them more stable under heat sterilization conditions. US 5,534,538 describes N-acyl dipeptides and their use in heat sterilized enteral or parenteral nutrition products.

Franek et al. (2002, Biotechnol. Prog. 18, 155-158; US 2004/0072341) screened various synthetic oligopeptides (including oligo-Gly, oligo-Ala) for their effect on a mouse cell line in serum-free medium. They reported that, while the single amino acid and the tested dipeptides did not significantly alter the culture performance, tri-, tetra- and penta-glycine, as well as tri- and tetra-alanine, enhanced viable cell density and cell viability.

WO 2011/133902 discloses cell culture media comprising dipeptides, wherein the dipeptides include amino acids having a low solubility in water, in this case tyrosine and cysteine. Cysteine does not only have a low solubility in water, but it is also unstable, due to the presence of reactive thiol group. The authors have found that by incorporation of tyrosine and cysteine in dipeptides, solubility and stability problems of the amino acids can be ameliorated. The authors also found an improved shelf life of the resulting cell culture media. In one embodiment, the remaining amino acid of the dipeptide (not cysteine or tyrosine) is asparagine.

WO 2012/019160 discloses animal cell cultures, wherein during the production phase the serum-free medium is supplemented with Tyr- and His-containing dipeptides. Positive effects of the addition of the Tyr- and His-containing dipeptides on growth and product formation are described.

EP 0220379 and DE3538310 disclose the use of glutamine-containing dipeptides and tripeptides in cell culture media. Glutamine is added in form of dipeptides, in order to avoid problems stemming from the instability of glutamine under elevated temperatures. The glutamine-containing dipeptides are used as a temperature insensitive glutamine source.

JP61271985 discloses a culture medium useful for animal cells including the dipeptides Xxx-L-glutamine, wherein Xxx is glycine, D/L-alanine, L-aspartic acid, L-glutamic acid, L-valine, L-leucine, L-serine, L-lysine or L-phenylalanine.

While cell culture media described in the above prior art provide satisfactory performance and properties for certain cell culture processes, the prior art culture media are still not optimal. There still exists a need for further improved culture media that promote better growth and product formation in biotechnological production processes.

### SUMMARY OF THE INVENTION

The above shortcomings of known culture media are addressed by the present invention. The invention is defined by the terms of the appended independent claims. Preferred embodiments of the invention are defined by the dependent claims.

The invention thus relates to a culture medium, preferably a cell culture medium, comprising at least one dipeptide, the dipeptide comprising asparagine (Asn) and glutamine (Gln), wherein said dipeptide is present in said culture medium at a concentration of from 0.1 to 10 mM, wherein the dipeptide is Gln-Asn.

The invention further relates to the use of a culture medium of the invention for culturing cells, preferably plant cells, animal cells or mammalian cells.

Another aspect of the invention relates to a method of manufacturing a cell culture product comprising the steps of (i) providing a cell capable of producing said cell culture product; (ii) contacting said cell with a culture medium according to the invention; and (iii) obtaining said cell culture product from said culture medium or from said cell.

Preferred embodiments of the invention are described in further detail in the following detailed description of the invention.

### BRIEF DESCRIPTION OF THE FIGURES

Figure 1 depicts the viable cell density over time in various cultures employing cell culture media with varying amounts of an dipeptide according to the invention.
Figure 2 depicts the cell viability over time in various cell cultures employing cell culture media with varying amounts of an dipeptide according to the invention.
Figure 3 depicts relative product concentrations obtained from cell cultures employing cell culture media with varying amounts of an dipeptide according to the invention.

### DETAILED DESCRIPTION OF THE INVENTION

A "culture medium", according to the invention, shall be understood as being a liquid or solid medium containing nutrients, the medium being suitable for nourishing and supporting life and/or product formation of cells in the culture. The cultured cells, according to the invention, may be bacterial cells, yeast cells, fungal cells, animal cells, such as mammalian cells or insect cells, and/or plant cells, e.g., algae. Typically, a culture medium provides essential and non-essential amino acids, vitamins, at least one energy source, lipids, and trace elements, all required by the cell for sustaining life, growth and/or product formation. The culture medium may also contain components that enhance growth and/or survival above the minimal rate, including hormones and growth factors. The culture medium has preferably a pH and a salt concentration which supports life, growth and/or product formation of the cells. A culture medium, according to the invention, preferably comprises all nutrients necessary to sustain life and proliferation of the cell culture. Preferred culture media are defined media.

A "defined medium", according to the invention is a medium that contain no cell extracts, cell hydrolysates, or protein hydrolysates. Preferred defined media comprise no components of unknown composition. As is commonly understood by the person skilled in the art, defined media are usually free of animal-derived components. Preferably, all components of a defined medium have a known chemical structure. Preferred defined media are serum-free media. Other preferred defined media are synthetic media. Culture media other than defined culture media are referred to as "complex" culture media.

A "cell culture medium" shall be understood as being a culture medium suitable for sustaining life, proliferation and/or product formation of animal cells and/or plant cells.

A "nutrient", according to the present invention, is a chemical compound or substance which is needed by cells to live and grow. The nutrient is preferably taken up by the cell from the environment. Nutrients can be "organic nutrients" and "inorganic nutrients". Organic nutrients include carbohydrates, fats, proteins (or their building blocks, e.g., amino acids), and vitamins. Inorganic nutrients are inorganic compounds such as, e.g., dietary minerals and trace elements. "Essential nutrients" are nutrients which the cell cannot synthesize itself, and which must thus be provided to the cell by the culture medium.

A "cell culture product", according to the invention, shall be understood as being any useful biological compound produced by cells in cell culture. Preferred cell culture products of the invention are therapeutic proteins, diagnostic proteins, therapeutic polysaccharides, such as heparin, antibodies, e.g., monoclonal antibodies, growth factors, interleukin, peptide hormones, and enzymes.

An "amino acid", in the context of the present invention, shall be understood as being a molecule comprising an amino functional group (-NHz) and a carboxylic acid functional group (-COOH), along with a side-chain specific to the respective amino acid. Preferred amino acids of the invention are alpha-amino acids, in particular the 20 "natural amino" acids as follows:

| | |
|---|---|
| Alanine | (Ala / A) |
| Arginine | (Arg / R) |
| Asparagine | (Asn / N) |
| Aspartic acid | (Asp / D) |
| Cysteine | (Cys / C) |
| Glutamic acid | (Glu / E) |
| Glutamine | (Gln / Q) |
| Glycine | (Gly / G) |
| Histidine | (His / H) |
| Isoleucine | (Ile / I) |
| Leucine | (Leu / L) |
| Lysine | (Lys / K) |
| Methionine | (Met / M) |
| Phenylalanine | (Phe / F) |
| Proline | (Pro / P) |
| Serine | (Ser / S) |
| Threonine | (Thr / T) |
| Tryptophan | (Trp / W) |
| Tyrosine | (Tyr / Y) |
| Valine | (Val/V) |

In the context of the present invention, the expression "natural amino acids" shall be understood to include both the L-form and the D-form of the above listed 20 amino acids. The L-form, however, is preferred. In one embodiment, the term "amino acid" also includes analogues or derivatives of those amino acids.

A "free amino acid", according to the invention, is understood as being an amino acid having its amino and its (alpha-) carboxylic functional group in free form, i.e., not covalently bound to other molecules, e.g., an amino acid not forming a peptide bond. Free amino acids may also be present as salts or in hydrate form. When referring to an amino acid as a part of, or in, an oligopeptide, this shall be understood as referring to that part of the respective oligopeptide structure derived from the respective amino acid, according to the known mechanisms of biochemistry and peptide biosynthesis.

A "growth factor", according to the invention, shall be understood as being any naturally occurring substance capable of stimulating cellular growth, proliferation and cellular differentiation. Preferred growth factors are in form of protein or steroid hormone. According to one embodiment of the invention, the expression "growth factor" shall be interpreted as relating to a growth factor selected from the list consisting of fibroblast growth factor (FGF), including acidic FGF and basic FGF, insulin, insulin-like growth factor (IGF), epithelial growth factor (EGF), nerve growth factor (NGF), platelet-derived growth factor (PDGF), and transforming growth factor (TGF), including TGFalpha and TGFbeta, cytokine, such as interleukins 1, 2, 6, granulocyte stimulating factor, and leukocyte inhibitory factor (LIF).

An "oligopeptide", according to the invention, shall be understood as being a peptide compound consisting of 2 to 20 amino acids. More preferred oligopeptides of the inventions are oligopeptides consisting of 2-10 amino acids, 2-6 amino acids, 2-5 amino acids, 2-4 amino acids, or 2-3 amino acids. Most preferred oligopeptides according to the invention are dipeptides.

A "peptide" shall be understood as being a molecule comprising at least two amino acids covalently coupled to each other by a peptide bond (R¹-CO-NH-R²).

The expression "Xxx", when used herein in connection with an amino acid, shall be understood as referring to any natural amino acid as listed hereinabove.

The expression "N-acylated", with reference to a chemical compound, such as an amino acid, shall be understood as meaning that the N-acylated compound is modified by the addition of an acyl group to a nitrogen functional group of said compound. Preferably, the acyl group is added to the alpha-amino group of the amino acid.

A "sterile form" of a nutrient composition, culture medium, cell culture medium, or the like, shall be understood as defining the absence of any living matter in said composition, culture medium, cell culture medium or the like.

A "solid culture medium", in the context of the present invention, shall be understood as being any non-liquid or non-gaseous culture medium. Preferred solid culture media of the invention are gel-like culture media, such as agar-agar, carrageen or gelatin.

"Passaging of cells" (also known as subculture or splitting of cells), in the context of the present invention, is understood as meaning the transferring a small number of cells into a new culture vessel. Cells can be cultured for a longer time if they are split regularly, as it avoids the senescence associated with prolonged high cell density. Suspension cultures are easily passaged with a small amount of culture containing a few cells diluted in a larger volume of fresh media.

The present invention generally relates to a cell culture medium, said culture medium comprising at least one dipeptide, the dipeptide comprising asparagine (Asn) and glutamine (Gln), wherein said dipeptide is present in the culture medium at a concentration of from 0.1 to 10 mM, wherein the dipeptide is Gln-Asn.

The present inventors found that culture media of this kind have superior properties over prior art cell culture media. It was found by the present inventors that the inventive culture media are capable of increasing the viable cell density and the cell viability of a cell culture, relative to a cell culture medium comprising the same amount of the relevant amino acids, however, in form of free amino acids. Despite the fact that the same amount of amino acids is present in the conventional cell culture medium, cell viability and cell number is increased when free amino acids are replaced by the Asn-containing dipeptides of the invention.

A preferred dipeptide of the invention is Gln-Asn (L-glutaminyl-L-asparagin).

In preferred embodiments, the dipeptide is not N-acylated. N-acylation is known to improve heat stability of certain dipeptides, however, it has been found that N-acylated dipeptides may also lead to inferior viable cell density and viability.

In one embodiment, the culture medium further comprises at least one carbohydrate, at least one free amino acid, at least one inorganic salt, a buffering agent and/or at least one vitamin. In a particularly preferred embodiment, the culture medium comprises all of at least one carbohydrate, at least one free amino acid, at least one inorganic salt, a buffering agent and at least one vitamin.

In one further embodiment of the invention, the culture medium does not contain a growth factor. In accordance with this embodiment, the dipeptide of the invention may be used instead of a growth factor for promoting growth and/or proliferation of the cells in culture. In another embodiment of the invention, the culture medium does not contain any lipids.

According to another embodiment of the invention, the culture medium is in liquid form, in form of a gel, a powder, a granulate, a pellet or in form of a tablet.

In preferred embodiments, the culture medium of the invention is a defined medium, or a serum-free medium. For example, dipeptides of the invention may be supplemented to the CHOMACS CD medium of Miltenyi Biotech (Bergisch Gladbach, Germany), to the PowerCHO-2 CD medium available from LONZA (Basel, Switzerland), the Acti-CHO P medium of PAA (PAA Laboratories, Pasching, Austria), the Ex-Cell CD CHO medium available from SAFC, the SFM4CHO medium and the CDM4CHO medium of ThermoFisher (Waltham, USA). The dipeptides of the invention may also be supplemented to DMEM medium (Life Technologies Corp., Carlsbad, USA). The invention, however, is not limited to supplementation of the above media.

In other preferred embodiments, the culture medium is a liquid medium in 2-fold, 3-fold, 3.33-fold, 4-fold, 5-fold or 10-fold concentrated form (volume/volume), relative to the concentration of said medium in use. This allows preparation of a "ready-to-use" culture medium by simple dilution of the concentrated medium with the respective volume of sterile water. Such concentrated forms of the medium of the invention may also be used by addition of the same to a culture, e.g., in a fed-batch cultivation process.

Culture media of the present invention preferably contain all nutrients required for sustained growth and product formation. Recipes for preparing culture media, in particular cell culture media, are well known to the person skilled in the art (see, e.g., Cell Culture Technology for Pharmaceutical and Cell-Based Therapies, Öztürk and Wei-Shou Hu eds., Taylor and Francis Group 2006). Various culture media are commercially available from various sources.

The culture media of the invention preferably include a carbohydrate source. The main carbohydrate used in cell culture media is glucose, routinely supplemented at 5 to 25 nM. In addition, any hexose, such as galactose, fructose, or mannose or a combination may be used.

The culture medium typically also includes at least the essential amino acids (i.e., His, Ile, Leu, Lys, Met, Phe, Thr, Try, Val) as well as certain non-essential amino acids. A non-essential amino acid is typically included in the cell culture medium if the cell line is not capable of synthesizing the amino acid or if the cell line cannot produce sufficient quantities of the amino acid to support maximal growth. In addition, mammalian cells can also use glutamine as a major energy source. Glutamine is often included at higher concentrations than other amino acids (2-8 mM). However, as noted above, glutamine can spontaneously break down to form ammonia and certain cell lines produce ammonia faster, which is toxic.

The culture media of the invention preferably comprise salts. Salts are added to cell culture medium to maintain isotonic conditions and prevent osmotic imbalances. The osmolality of a culture medium of the invention is about 300 mOsm/kg, although many cell lines can tolerate an approximately 10 percent variation of this value or higher. The osmolality of some insect cell cultures tend to be higher than 300 mOsm/kg, and this may be 0.5 percent, 1 percent, 2 to 5 percent, 5- 10 percent, 10-15 percent, 15- 20 percent, 20-25 percent, 25-30 percent higher than 300 mOsm/kg. The most commonly used salts in cell culture medium include Na⁺, K⁺, Mg²⁺, Ca²⁺, Cl⁻, SO₄²⁻, PO₄³⁻, and HCO₃⁻ (e.g., CaCl₂, KCI, NaCl, NaHCOs, Na₂HPO₄).

Other inorganic elements may be present in the culture medium. They include Mn, Cu, Zn, Mo, Va, Se, Fe, Ca, Mg, Si, and Ni. Many of these elements are involved in enzymatic activity. They may be provided in the form of salts such as CaCl₂, Fe(NO₃)₃, MgCl₂, MgSO₄, MnCl₂, NaCl, NaHCOs, Na₂HPO₄, and ions of the trace elements, such as, selenium, vanadium and zinc. These inorganic salts and trace elements may be obtained commercially, for example from Sigma (Saint Louis, Missouri).

The culture media of the invention preferably comprise vitamins. Vitamins are typically used by cells as cofactors. The vitamin requirements of each cell line vary greatly, although generally extra vitamins are needed if the cell culture medium contains little or no serum or if the cells are grown at high density. Exemplary vitamins preferably present in culture media of the invention include biotin, choline chloride, folic acid, i-inositol, nicotinamide, D-Ca⁺⁺-pantothenate, pyridoxal, riboflavin, thiamine, pyridoxine, niacinamide, A, B₆, B₁₂, C, D₃, E, K, and p-aminobenzoic acid (PABA).

Culture media of the invention may also comprise serum. Serum is the supernatant of clotted blood. Serum components include attachment factors, micronutrients (e.g., trace elements), growth factors (e.g., hormones, proteases), and protective elements (e.g., antitoxins, antioxidants, antiproteases). Serum is available from a variety of animal sources including human, bovine or equine serum. When included in cell culture medium according to the invention, serum is typically added at a concentration of 5-10 %(vol.). Preferred cell culture media are serum-free.

To promote cell growth in the absence or serum or in serum reduced media, one or more of the following polypeptides can be added to a cell culture medium of the invention: for example, fibroblast growth factor (FGF), including acidic FGF and basic FGF, insulin, insulin-like growth factor (IGF), epithelial growth factor (EGF), nerve growth factor (NGF), platelet-derived growth factor (PDGF), and transforming growth factor (TGF), including TGFalpha and TGFbeta, any cytokine, such as interleukins 1, 2, 6, granulocyte stimulating factor, leukocyte inhibitory factor (LIF), etc.

However, the culture medium of the invention may also include none of the above listed growth factors. According to this aspect of the invention, the dipeptide of the invention is used instead of any growth factor for promoting proliferation of the cells. In other words, according to this aspect of the invention, the presence of the dipeptide of the invention makes the presence of a growth factor dispensable.

In other embodiments, the cell culture medium does not comprise polypeptides (i.e., peptides with more than 20 amino acids).

One or more lipids can also be added to a cell culture medium of the invention, such as linoleic acid, linolenic acid, arachidonic acid, palmitoleic acid, oleic acid, polyenoic acid, and/or fatty acids of 12, 14, 16, 18, 20, or 24 carbon atoms, each carbon atom branched or unbranched), phospholipids, lecithin (phophatidylcholine), and cholesterol. One or more of these lipids can be included as supplements in serum-free media. Phosphatidic acid and lysophosphatidic acid stimulate the growth of certain anchorage-dependent cells, such as MDCK, mouse epithelial, and other kidney cell lines, while phosphatidylcholine, phosphatidylethanolamine, and phosphatidylinositol stimulate the growth of human fibroblasts in serum-free media. Ethanolamine and cholesterol have also been shown to promote the growth of certain cell lines. In certain embodiment, the cell culture medium does not contain a lipid.

One or more carrier proteins, such as bovine serum albumin (BSA) or transferrin, can also be added to the cell culture medium. Carrier proteins can help in the transport of certain nutrients or trace elements. BSA is typically used as a carrier of lipids, such as linoleic and oleic acids, which are insoluble in aqueous solution. In addition, BSA can also serve as a carrier for certain metals, such as Fe, Cu, and Ni. In protein-free formulations, non-animal derived substitutes for BSA, such as cyclodextrin, can be used as lipid carriers.

One or more attachment proteins, such as fibronectin, laminin, and pronectin, can also be added to a cell culture medium to help promote the attachment of anchorage-dependent cells to a substrate.

The cell culture medium can optionally include one or more buffering agents. Suitable buffering agents include, but are not limited to, N-[2-hydroxyethyl]-piperazine- N'-[2-ethanesulfonic acid] (HEPES), MOPS, MES, phosphate, bicarbonate and other buffering agents suitable for use in cell culture applications. A suitable buffering agent is one that provides buffering capacity without substantial cytotoxicity to the cells cultured. The selection of suitable buffering agents is within the ambit of ordinary skill in the art of cell culture.

Polyanionic or polycationic compounds may be added to the culture medium to prevent the cells from clumping and to promote growth of the cells in suspension.

In a preferred embodiment, the culture medium is in liquid form. The culture medium, however, can also be a solid medium, such as a gel-like medium, e.g. an agar-agar-, carrageen- or gelatin-containing medium.

Preferably, the culture medium is in sterile form.

In preferred embodiments of the invention, the Asn- and Gln-containing dipeptide is present in said culture medium in a concentration of from 0.01 to 20 g/l, or 0.1 to 10 g/l, or 0.5 to 5 g/l. In other preferred embodiments of the invention, the Asn- and Gln-containing dipeptide is present in a concentration of above 0.2, 0.4, or 1 mM. Also preferred are culture media in which the Asn- and Gln-containing dipeptide is present in a concentration of less than 5, or 2 mM. Preferably, the Asn- and Gln-containing dipeptide is present in the medium at a concentration of from 0.5 mM to 10 mM, or 1 mM to 10 mM, or 1 mM to 8 mM, or 1 mM to 6 mM. In one very preferred embodiment, the concentration of the dipeptide is from 1 mM to 6 mM, and the dipeptide is Gln-Asn.

The above concentrations are given as concentrations in the non-concentrated medium, i.e., the concentration as present in the actual culture. Concentrated media may include X-fold higher concentrations.

In certain embodiments the cell culture medium of the invention comprises both Asn- and Gln-containing dipeptides and free Asn. In one preferred embodiment, the Asn- and Gln-containing dipeptide is added to a commercially available culture medium, e.g., to a defined cell culture medium or to a complex cell culture medium.

The culture medium of the present invention can be in concentrated form. It may be, e.g., in 2-fold, 3-fold, 3.33-fold, 4-fold, 5-fold, 10-fold, 20-fold, 50-fold or 100-fold concentrated form (relative to a concentration that supports growth and product formation of the cells). Such concentrated culture media are helpful for preparing the culture medium for use by dilution of the concentrated culture medium with an aqueous solvent, such as water. Such concentrated culture media may be used in batch culture, but are also advantageously used in fed-batch or continuous cultures, in which a concentrated nutrient composition is added to an ongoing cultivation of cells, e.g., to replenish nutrients consumed by the cells during culture.

In other embodiments of the invention, the culture medium is in dry form, e.g., in form of a dry powder, or in form of granules, or in form of pellets, or in form of tablets.

The present invention also relates to the use of a culture medium of the invention for culturing cells. Another aspect of the invention relates to the use of a culture medium of the invention for producing a cell culture product.

A preferred embodiment of the invention relates to the use of a culture medium according to the invention for culturing animal cells or plant cells, most preferred mammalian cells. In specific embodiments the cells to be cultured are CHO cells, COS cells, VERO cells, BHK cells, HEK cells, HELA cells, AE-1 cells, insect cells, fibroblast cells, muscle cells, nerve cells, stem cells, skin cells, endothelial cells and hybridoma cells. Preferred cells of the invention are CHO cells and hybridoma cells. Most preferred cells of the invention are CHO cells. Particularly preferred CHO cells of the invention are CHO DG44 and CHO DP12 cells.

Also included in the scope of the present invention is a method of culturing cells, said method comprising contacting said cells with a cell culture medium according to the invention. In one embodiment of the invention, the method of culturing cells comprises contacting the cell with a basal culture medium under conditions supporting the cultivation of the cell and supplementing the basal cell culture medium with a concentrated medium according to the present invention. In preferred embodiments, the basal culture medium is supplemented with the concentrated feed or medium on more than one day.

Another aspect of the invention relates to a method of producing a culture medium according to the invention, wherein said culture medium comprises at least one dipeptide according to the invention. Methods of producing a culture medium according to the invention comprise at least one step of adding an dipeptide of the invention to the culture medium. Likewise, an aspect of the invention relates to the use of an Asn-containing dipeptide of the invention for producing a cell culture medium.

Another aspect of the invention relates to a method of modifying a culture medium, wherein said modifying of said culture medium comprises addition of at least one dipeptide of the invention to said culture medium.

Another aspect of the invention relates to a method of producing a liquid culture medium, said method comprising providing solid medium according to the invention, e.g., in form of a dry powder, or in form of granules, or in form of pellets, or in form of tablets; and dissolving said solid culture medium in an aqueous medium, such as water.

The invention also relates to methods of manufacturing a cell culture product comprising the steps of (i) providing a cell capable of producing said cell culture product; (ii) contacting said cell with a culture medium of the invention; and (iii) obtaining said cell culture product from said culture medium or from said cell. Likewise, the present invention relates to the use of an dipeptide according to the invention for manufacturing a cell culture product.

In preferred methods, the cell culture product is a therapeutic protein, a diagnostic protein, a polysaccharide, such as heparin, an antibody, a monoclonal antibody, a growth factor, an interleukin, virus, virus-like particle or an enzyme

In a preferred method of cultivating cells, or use of an dipeptide for the cultivation of cells, according to the invention, the dipeptide is Gln-Asn, the dipeptide is present at a concentration of from 1 to 6 mM, and the cell is a CHO cell. In another preferred method of cultivating cells, or use of an dipeptide for the cultivation of cells, according to the invention, the dipeptide is Gln-Asn, the dipeptide is present at a concentration of from 1 to 6 mM, and the cell is a CHO cell or a hybridoma cell. In another preferred method of cultivating cells, or use of an dipeptide for the cultivation of cells, according to the invention, the dipeptide is Gln-Asn, the dipeptide is present at a concentration of from 1 to 6 mM, and the cell is a mammalian cell. In another preferred method of cultivating cells, or use of an dipeptide for the cultivation of cells, according to the invention, the dipeptide is Gln-Asn, the dipeptide is present at a concentration of from 0.5 to 10 mM, and the cell is a CHO cell. In another preferred method of cultivating cells, or use of an dipeptide for the cultivation of cells, according to the invention, the dipeptide is Gln-Asn, the dipeptide is present at a concentration of from 0.5 to 10 mM, and the cell is a CHO cell or a hybridoma cell. In another preferred method of cultivating cells, or use of an dipeptide for the cultivation of cells, according to the invention, the dipeptide is Gln-Asn, the dipeptide is present at a concentration of from 0.5 to 10 mM, and the cell is a mammalian cell. In another preferred method of cultivating cells, or use of an dipeptide for the cultivation of cells, according to the invention, the dipeptide is Gln-Asn, the dipeptide is present at a concentration of from 0.1 to 20 mM, and the cell is a CHO cell. In another preferred method of cultivating cells, or use of an dipeptide for the cultivation of cells, according to the invention, the dipeptide is Gln-Asn, the dipeptide is present at a concentration of from 0.1 to 20 mM, and the cell is a CHO cell or a hybridoma cell. In another preferred method of cultivating cells, or use of an dipeptide for the cultivation of cells, according to the invention, the dipeptide is Gln-Asn, the dipeptide is present at a concentration of from 0.1 to 10 mM, and the cell is a mammalian cell. In another preferred method of cultivating cells, or use of an dipeptide for the cultivation of cells, according to the invention, the dipeptide is Gln-Asn, the dipeptide is present at a concentration of from 1 to 6 mM, and the cell is a CHO cell. In another preferred method of cultivating cells, or use of an dipeptide for the cultivation of cells, according to the invention, the dipeptide is Gln-Asn, the dipeptide is present at a concentration of from 1 to 6 mM, and the cell is a CHO cell or a hybridoma cell. In another preferred method of cultivating cells, or use of an dipeptide for the cultivation of cells, according to the invention, the dipeptide is Gln-Asn or Asn-Gln, the dipeptide is present at a concentration of from 1 to 6 mM, and the cell is a mammalian cell. In another preferred method of cultivating cells, or use of an dipeptide for the cultivation of cells, according to the invention, the dipeptide is Gln-Asn, the dipeptide is present at a concentration of from 0.5 to 10 mM, and the cell is a CHO cell. In another preferred method of cultivating cells, or use of an dipeptide for the cultivation of cells, according to the invention, the dipeptide is Gln-Asn, the dipeptide is present at a concentration of from 0.5 to 10 mM, and the cell is a CHO cell or a hybridoma cell. In another preferred method of cultivating cells, or use of an dipeptide for the cultivation of cells, according to the invention, the dipeptide is Gln-Asn, the dipeptide is present at a concentration of from 0.5 to 10 mM, and the cell is a mammalian cell. In another preferred method of cultivating cells, or use of an dipeptide for the cultivation of cells, according to the invention, the dipeptide is Gln-Asn, the dipeptide is present at a concentration of from 0.1 to 10 mM, and the cell is a CHO cell. In another preferred method of cultivating cells, or use of an dipeptide for the cultivation of cells, according to the invention, the dipeptide is Gln-Asn, the dipeptide is present at a concentration of from 0.1 to 20 mM, and the cell is a CHO cell or a hybridoma cell. In another preferred method of cultivating cells, or use of an dipeptide for the cultivation of cells, according to the invention, the dipeptide is Gln-Asn, the dipeptide is present at a concentration of from 0.1 to 10 mM, and the cell is a mammalian cell. In another preferred method of cultivating cells, or use of an dipeptide for the cultivation of cells, according to the invention, the dipeptide Gln-Asn is present at a concentration of from 1 to 6 mM, and the cell is a CHO cell. In another preferred method of cultivating cells, or use of an dipeptide for the cultivation of cells, according to the invention, the dipeptide Gln-Asn is present at a concentration of from 1 to 6 mM, and the cell is a CHO cell or a hybridoma cell. In another preferred method of cultivating cells, or use of an dipeptide for the cultivation of cells, according to the invention, the dipeptide Gln-Asn is present at a concentration of from 1 to 6 mM, and the cell is a mammalian cell. In another preferred method of cultivating cells, or use of an dipeptide for the cultivation of cells, according to the invention, the dipeptide Gln-Asn is present at a concentration of from 0.5 to 10 mM, and the cell is a CHO cell. In another preferred method of cultivating cells, or use of an dipeptide for the cultivation of cells, according to the invention, the dipeptide Gln-Asn is present at a concentration of from 0.5 to 10 mM, and the cell is a CHO cell or a hybridoma cell. In another preferred method of cultivating cells, or use of an dipeptide for the cultivation of cells, according to the invention, the dipeptide Gln-Asn is present at a concentration of from 0.5 to 10 mM, and the cell is a mammalian cell. In another preferred method of cultivating cells, or use of an dipeptide for the cultivation of cells, according to the invention, the dipeptide Gln-asn is present at a concentration of from 0.1 to 10 mM, and the cell is a CHO cell. In another preferred method of cultivating cells, or use of an dipeptide for the cultivation of cells, according to the invention, the dipeptide Gln-Asn present at a concentration of from 0.1 to 10 mM, and the cell is a CHO cell or a hybridoma cell. In another preferred method of cultivating cells, or use of an dipeptide for the cultivation of cells, according to the invention, the dipeptide Gln-Asn is present at a concentration of from 0.1 to 10 mM, and the cell is a mammalian cell.

Cultivation of cells, according to the invention can be performed in batch culture, in fed-batch culture or in continuous culture.

### EXAMPLES

### Example 1: Synthesis of L-glutaminyl-L-asparagine

27.8 g triethylamine was added to 70 g benzyloxycarbonyl-L-glutamine (CAS Registry Number 2650-64-8, available from Peptides International, Inc., USA) in 350 ml acetone. The solution was cooled to -10°C and 31.6 g pivaloyl chloride (Sigma) was added. The mixture aws stirred for 10 min and was then added to a solution of 39.6 g L-asparagine, 19.0 g potassium hydroxide 85 % and 41.3 g potassium carbonate in 350 ml water. Heated to room temperature (20°C) and diluted with 300 ml H₂O. HCl (conc.) was added to pH 2. The obtained solid was filtrated, washed with water and acetone and dried under vacuum. 65.2 g of benzyloxycarbonyl-L-glutaminyl-L-asparagine was obtained in form of colorless crystals. The melting point was 202-204°C.

¹H-NMR (DMSO): 1.69 (m, 1H), 1.92 (m, 1H), 2.14 (m, 2H), 2.54 (m, 2H), 4.02 (m, 1H), 4.52 (m, 1H), 5.03 (s, 2H), 6.64 (d, 2H), 7.28 (d, 2H), 7.37 (m, 5H), 7.43 m, 1H), 8.09 (m, 1H).

20.0 g of the benzyloxycarbonyl-L-glutaminyl-L-asparagine was then suspended in 500 ml water. 1.0 g Pd/C catalyst (5% Pd loading) was added and hydrated for 14 hours at 20-30°C and 5 bar. Heat was applied to 40-45°C and catalyst was removed by filtration. The filtrate was concentrated under vacuum in a rotary evaporator. The remainder was mixed with 100 ml EtOH. The obtained solid was filtrated, washed with EtOH and dried under vacuum. 12.9 g of L-glutaminyl-L-asparagine was obtained in form of colourless crystals. Melting point was 202-204°C.

¹H-NMR (DMSO + HCl): 2.05 (m, 2H), 2.42 (m, 2H), 2.69 (m, 2H), 3.94 (m, 1H), 4.62 (m, 1H), 8.46 (s, 3H), 8.96 m, 1H).

### Example 2: Effect of Gln-Asn on cell culture

The effect of the Gln-Asn dipeptide on an antibody-producing Chinese Hamster Ovary (CHO) cell (subclone DG44; Life Technologies Corporation, Carlsbad, USA) was investigated using one-factor-at-a-time (OFAT) analysis.

The analysis was based on a comparison of the viable cell density and cell viability in a series of cell cultures, which were conducted at substantially identical culturing conditions, however, with increasing amounts of the Gln-Asn dipeptide in the medium. In order to keep the total amounts of Asn and Gln units (molar basis) in the various culture media constant, the molar concentration of free glutamine and free asparagine was reduced in accordance with the molar amount of the dipeptide added. A series of culture media having increasing amounts of the Gln-Asn dipeptide was prepared by mixing varying amounts of a Base Medium (comprising free Asn and Gln) and a Test Medium (comprising Gln-Asn dipeptide). A commercially available defined cell culture medium (TC-42, TeutoCell AG, Bielefeld, Germany) served as the Base Medium. (Any other suitable cell culture medium could be used.) A Test Medium (TC-42-EVO) was prepared so as to have identical nutrient concentrations compared to the Base Medium, however, instead of free Asn the Test Medium contained an equimolar amount (5.4 mM) of Gln-Asn dipeptide. Furthermore, the free Gln concentration in the Test Medium was set to a value such that the total concentration of Gln units in the Test Medium (i.e., free Gln and dipeptide-Gln) matched the concentration of free Gln in the Base Medium. All other nutrient concentrations in the Test Medium were the same as in the Base Medium. The Base Medium contained 1461.5 mg/l free Gln and 612 mg/l free Asn.

Six cell cultivations were then conducted with media containing varying amounts of the Test Medium and the Base Medium in the following proportions:

**Table 1:**

| Cultivation | Base Medium [%] (free Asn + Gln) | Test Medium [%] (Gln-Asn dipeptide) | [Gln-Asn] mM |
|---|---|---|---|
| #1 | 0 | 100 | 5.4 |
| #2 | 20 | 80 | 4.3 |
| #3 | 75 | 25 | 1.4 |
| #4 | 85 | 15 | 0.8 |
| #5 | 95 | 5 | 0.3 |
| #6 | 100 | 0 | 0.0 |

All media were set to pH 8.3. Salinity of the culture media was set to 285 mOsmol/kg with NaCl. All cultivations were conducted in on a rotary shaker under identical culture conditions (temperature, humidity, rpm, CO₂, etc.), using standard cell culture methods. Three passages of a cell culture are conducted and viable cell density (VCD; 10⁶ viable cells/ml) and cell viability (%) was followed in duplicate determinations, using tryptophane blue determination.

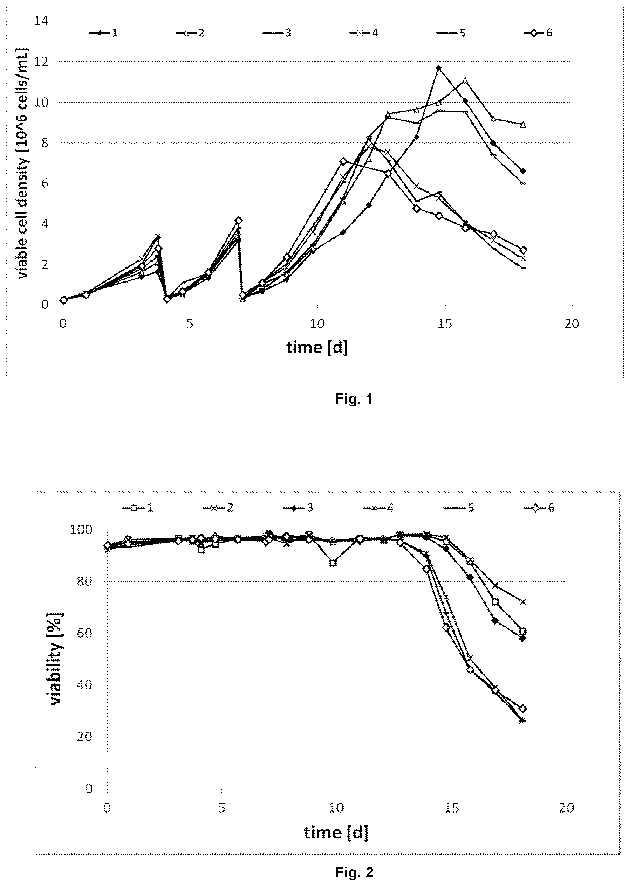

The viable cell density [10⁶ cells/ml] in cultivation #1-6 is shown in Figure 1 and Table 2, below. A correlation of the maximum viable cell density (third passage) with the concentration of the dipeptide is observed. The greatest maximum viable cell density is obtained in cultivation #1, i.e., in the cultivation in which all Asn is provided in the form of the Asn-containing dipeptide. The lowest maximum viable cell density is obtained in cultivation #6, i.e., in the cultivation in which all of the Asn in provided as free amino acid. Furthermore, it can be seen that the increased viable cell density is maintained over an extended period of time (day 13 through day 18). A positive effect of Asn-containing dipeptides on the viable cell density is thus demonstrated.

**Table 2:**

| **time [h]** | **#1** | **#2** | **#3** | **#4** | **#5** | **#6** |
|---|---|---|---|---|---|---|
| 0 | 0,28 | 0,27 | 0,265 | 0,27 | 0,285 | 0,265 |
| 0,89 | 0,59 | 0,51 | 0,495 | 0,565 | 0,485 | 0,5 |
| 3,08 | 1,38 | 1,615 | 1,8 | 2,285 | 1,985 | 1,93 |
| 3,71 | 1,64 | 2,13 | 2,415 | 3,4 | 3,325 | 2,8 |
| 4,08 | 0,365 | 0,3 | 0,315 | 0,365 | 0,265 | 0,3 |
| 4,70 | 0,56 | 0,54 | 0,65 | 0,665 | 1,12 | 0,665 |
| 5,69 | 1,33 | 1,545 | 1,47 | 1,545 | 1,555 | 1,615 |
| 6,88 | 3,16 | 3,555 | 3,365 | 3,36 | 3,8 | 4,175 |
| 7,04 | 0,335 | 0,305 | 0,33 | 0,32 | 0,31 | 0,505 |
| 7,80 | 0,665 | 1,045 | 0,77 | 1,12 | 1,17 | 1,1 |
| 8,78 | 1,26 | 1,545 | 1,615 | 1,86 | 2,01 | 2,375 |
| 9,82 | 2,67 | 2,8 | 2,97 | 3,6 | 3,9 | - |
| 10,99 | 3,57 | 5,115 | 5,25 | 6,31 | 6,065 | 7,11 |
| 12,02 | 4,91 | 7,215 | 8,29 | 7,805 | 8,21 | - |
| 12,75 | 6,415 | 9,42 | 9,23 | 7,545 | 7,135 | 6,52 |
| 13,89 | 8,285 | 9,645 | 8,975 | 5,86 | 5,13 | 4,77 |
| 14,74 | 11,695 | 9,985 | 9,58 | 5,26 | 5,54 | 4,4 |
| 15,79 | 10,075 | 11,08 | 9,535 | 4,065 | 4,075 | 3,81 |
| 16,89 | 7,995 | 9,195 | 7,395 | 3,175 | 2,775 | 3,505 |
| 18,06 | 6,6 | 8,905 | 5,985 | 2,3 | 1,825 | 2,725 |

Cell viability [% viable cells in total cells] over cultivation time is shown in Figure 2 and Table 3, below. Cultivations conducted with culture media containing high concentrations of the Asn-containing dipeptide (cultivations #1, #2, #3) show a relatively high viability of the cells at day 18, whereas cultivations #4, #5 and #6 (having a lower concentration of the dipeptide) show a significantly reduced cell viability at the end of experiment. Culture media of the invention thus have positive effect on the cell viability [%] in cell culture.

**Table 3:**

| **time** | **#1** | **#2** | **#3** | **#4** | **#5** | **#6** |
|---|---|---|---|---|---|---|
| 0 | 93,8 | 93,45 | 92,85 | 92,25 | 93,55 | 94,2 |
| 0,89 | 96,3 | 96,2 | 95,25 | 94,35 | 93,3 | 94,8 |
| 3,08 | 96,65 | 96,25 | 96,15 | 95,7 | 95,95 | 95,8 |
| 3,71 | 95,85 | 97,1 | 95,75 | 96,55 | 96,05 | 96,25 |
| 4,08 | 92,35 | 95,05 | 95,5 | 96,35 | 95,35 | 96,9 |
| 4,70 | 94,65 | 96,65 | 97,8 | 97,05 | 95,8 | 96,6 |
| 5,69 | 96,55 | 97,05 | 96,1 | 96,65 | 96 | 96,25 |
| 6,88 | 97 | 97,45 | 96,6 | 97,05 | 96,55 | 95,65 |
| 7,04 | 98,6 | 97,1 | 96,45 | 97,95 | 98,85 | 96,3 |
| 7,80 | 96,35 | 94,7 | 97,95 | 96,2 | 95,55 | 97,4 |
| 8,78 | 98,25 | 98 | 97,1 | 95,95 | 96,55 | 96,4 |
| 9,82 | 87,3 | 95,1 | 95,8 | 95,85 | 95,35 | - |
| 10,99 | 96,9 | 97,05 | 95,65 | 96,6 | 96,85 | 96,45 |
| 12,02 | 96,1 | 96,3 | 96,65 | 96,8 | 96,35 | - |
| 12,75 | 98,2 | 98,3 | 97,95 | 95,9 | 96,05 | 95,15 |
| 13,89 | 97,8 | 98,5 | 97,35 | 90,85 | 90 | 84,85 |
| 14,74 | 95,65 | 97,15 | 92,5 | 73,95 | 67,85 | 62,2 |
| 15,79 | 87,8 | 88,6 | 81,5 | 50,3 | 46,05 | 46,1 |
| 16,89 | 72,1 | 78,45 | 64,85 | 39,05 | 37,2 | 38,05 |
| 18,06 | 60,95 | 72,25 | 58,15 | 26,45 | 26,1 | 30,9 |

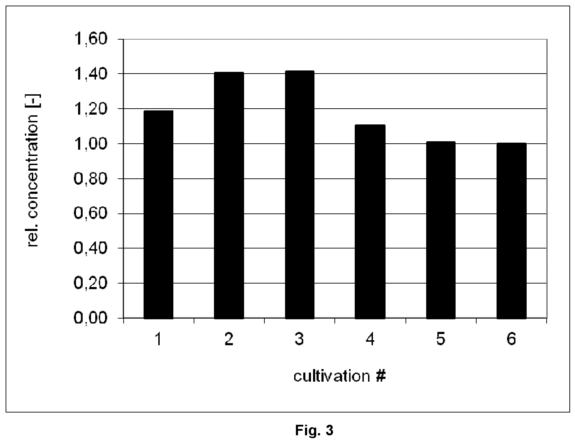

The product titer (antibody concentration at the end of the cultivation, t = 18h; in relative units) is shown in Figure 3 and Table 4, below. Generally, an increased product titer is observed in all cultures including the Asn-containing dipeptide. In cultivations #2 and #3 (80% and 25% of the Asn present in form of the dipeptide, respectively) the product titer is more than 1.4-fold increased. Significantly improved product titers are obtained in cultivations #1-#4, i.e., at molar concentrations of the dipeptide of from 0.8 mM to 5.4 mM, and above.

**Table 4:**

| **Cultivation** | **rel. product titer [-]** |
|---|---|
| #1 | 1,19 |
| #2 | 1,41 |
| #3 | 1,42 |
| #4 | 1,11 |
| #5 | 1,01 |
| #6 | 1,00 |

### Example 3: Preparation of a modified DMEM medium comprising an Asn-containing dipeptide

Dulbecco's Modified Eagle Medium (DMEM) is a widely used basal medium for supporting the growth of many different mammalian cells. Cells successfully cultured in DMEM include primary fibroblasts, neurons, glial cells, HUVECs and smooth muscle cells, as well as cell lines such as HeLa, 293, Cos-7, and PC-12. A supplemented DMEM medium according to the invention is prepared by adding Gln-Asn to commercially available DMEM medium (Gibco^{(R)} DMEM, High Glucose, SKU#41965-039, Life Technologies Corp., Carlsbad, CA, USA). The final concentration of the dipeptide in the medium is 4.5 mM.

### Example 4: Preparation of a modified DMEM Medium comprising a Asn-containing pentapeptide

DMEM medium (Gibco^{(R)} DMEM, High Glucose, SKU#41965-039, Life Technologies Corp., Carlsbad, CA, USA) is supplemented with a Gln-Phe-Asn-Gln-Asn pentapeptide. The final concentration of the pentapeptide is 4.5 mM.

## Claims

1. A culture medium, such as a cell culture medium, comprising at least one dipeptide, the dipeptide comprising asparagine (Asn) and glutamine (Gln), wherein said dipeptide is present in said culture medium at a concentration of from 0.1 to 10 mM, wherein the dipeptide is Gln-Asn.

2. The culture medium of any one of the preceding claims, said culture medium further comprising at least one carbohydrate, at least one free amino acid, at least one inorganic salt, a buffering agent and/or at least one vitamin.

3. The culture medium of any one of the preceding claims, wherein said medium does not contain a growth factor.

4. The culture medium of any one of the preceding claims, wherein said culture medium is in liquid form, in form of a gel, a powder, a granulate, a pellet or in form of a tablet.

5. The culture medium of any one of the preceding claims, wherein said cell culture medium is a serum-free medium, or a defined medium.

6. The culture medium of any one of the preceding claims, wherein said culture medium is in 2-fold, 3-fold, 3.33-fold, 4-fold, 5-fold or 10-fold concentrated form, relative to the concentration of said medium in use.

7. Use of a culture medium of any one of claims 1-6 for culturing cells.

8. Use of claim 7, wherein said cells are animal cells, such as mammalian cells, or plant cells.

9. Use of claim 8, wherein said cells are selected from the list consisting of CHO cells, COS cells, VERO cells, BHK cells, HEK cells, HELA cells, AE-1 cells, insect cells, fibroblast cells, muscle cells, nerve cells, stem cells, skin cells, endothelial cells and hybridoma cells.

10. Method of manufacturing a cell culture product comprising the steps of
- providing a cell capable of producing said cell culture product;
- contacting said cell with a culture medium of any one of claims 1-6; and
- obtaining said cell culture product from said culture medium or from said cell.

11. Method of claim 10, wherein said cell culture product is selected from the group consisting of therapeutic protein, diagnostic protein, polysaccharide, such as heparin, antibody, monoclonal antibody, growth factor, interleukin, virus, virus-like particle and enzyme.

## Patentansprüche

1. Kulturmedium, wie ein Zellkulturmedium, umfassend wenigstens ein Dipeptid, wobei das Dipeptid Asparagin (Asn) und Glutamin (Gln) umfasst, wobei das Dipeptid in dem Kulturmedium in einer Konzentration von 0,1 bis 10 mM vorhanden ist, wobei das Dipeptid Gln-Asn ist.

2. Kulturmedium nach einem der vorhergehenden Ansprüche, wobei das Kulturmedium ferner wenigstens ein Kohlenhydrat, wenigstens eine freie Aminosäure, wenigstens ein anorganisches Salz, eine Puffersubstanz und/oder wenigstens ein Vitamin umfasst.

3. Kulturmedium nach einem der vorhergehenden Ansprüche, wobei das Medium keinen Wachstumsfaktor enthält.

4. Kulturmedium nach einem der vorhergehenden Ansprüche, wobei das Kulturmedium in flüssiger Form, in Form eines Gels, eines Pulvers, eines Granulats, eines Pellets oder in Form einer Tablette vorliegt.

5. Kulturmedium nach einem der vorhergehenden Ansprüche, wobei das Zellkulturmedium ein serumfreies Medium oder ein definiertes Medium ist.

6. Kulturmedium nach einem der vorhergehenden Ansprüche, wobei das Kulturmedium in 2-fach, 3-fach, 3,33-fach, 4-fach, 5-fach oder 10-fach konzentrierter Form im Vergleich zu der Konzentration des Mediums bei der Verwendung vorliegt.

7. Verwendung eines Kulturmediums nach einem der Ansprüche 1-6 für das Kultivieren von Zellen.

8. Verbindung nach Anspruch 7, wobei die Zellen Tierzellen, wie Säugetierzellen, oder Pflanzenzellen sind.

9. Verbindung nach Anspruch 8, wobei die Zellen aus der Liste bestehend aus CHO-Zellen, COS-Zellen, VERO-Zellen, BHK-Zellen, HEK-Zellen, HELA-Zellen, AE-1-Zellen, Insektenzellen, Fibroblastenzellen, Muskelzellen, Nervenzellen, Stammzellen, Hautzellen, Endothelialzellen und Hybridomazellen ausgewählt sind.

10. Verfahren zur Herstellung eines Zellkulturprodukts, umfassend die folgenden Schritte:
- Bereitstellen einer Zelle, die zur Herstellung des Zellkulturprodukts in der Lage ist;
- Kontaktieren der Zelle mit einem Kulturmedium nach einem der Ansprüche 1-6; und
- Gewinnen des Zellkulturprodukts aus dem Kulturmedium oder aus der Zelle.

11. Verfahren nach Anspruch 10, wobei das Zellkulturprodukt aus der Gruppe bestehend aus therapeutischem Protein, diagnostischem Protein, Polysaccharid, wie Heparin, Antikörper, monoklonalem Antikörper, Wachstumsfaktor, Interleukin, Virus, virusartigem Partikel und Enzym ausgewählt wird.

## Revendications

1. Milieu de culture, tel qu'un milieu de culture cellulaire, comprenant au moins un dipeptide, le dipeptide comprenant l'asparagine (Asn) et la glutamine (Gin), ledit dipeptide étant présent dans ledit milieu de culture en une concentration allant de 0,1 à 10 mM, le dipeptide étant Gln-Asn.

2. Milieu de culture selon la revendication précédente, ledit milieu de culture comprenant en outre au moins un glucide, au moins un acide aminé libre, au moins un sel inorganique, un agent tampon et/ou au moins une vitamine.

3. Milieu de culture selon l'une quelconque des revendications précédentes, ledit milieu ne contenant pas un facteur de croissance.

4. Milieu de culture selon l'une quelconque des revendications précédentes, ledit milieu de culture étant sous forme liquide, sous forme d'un gel, d'une poudre, d'un granulé, d'une pastille ou sous forme d'un comprimé.

5. Milieu de culture selon l'une quelconque des revendications précédentes, ledit milieu de culture cellulaire étant un milieu exempt de sérum, ou un milieu défini.

6. Milieu de culture selon l'une quelconque des revendications précédentes, ledit milieu de culture étant sous forme 2 fois, 3 fois, 3,33 fois, 4 fois, 5 fois ou 10 fois concentrée, par rapport à la concentration dudit milieu lors d'une utilisation.

7. Utilisation d'un milieu de culture selon l'une quelconque des revendications 1 à 6 pour la culture de cellules.

8. Utilisation selon la revendication 7, lesdites cellules étant des cellules animales, telles que des cellules de mammifère, ou des cellules végétales.

9. Utilisation selon la revendication 8, lesdites cellules étant choisies dans la liste constituée par les cellules CHO, les cellules COS, les cellules VERO, les cellules BHK, les cellules HEK, les cellules HELA, les cellules AE-1, les cellules d'insectes, les cellules de fibroblastes, les cellules musculaires, les cellules nerveuses, les cellules souches, les cellules cutanées, les cellules endothéliales et les cellules d'hybridomes.

10. Procédé de fabrication d'un produit de culture cellulaire comprenant les étapes de
- mise à disposition d'une cellule capable de produire ledit produit de culture cellulaire ;
- mise en contact de ladite cellule avec un milieu de culture selon l'une quelconque des revendications 1 à 6 ; et
- obtention dudit produit de culture cellulaire depuis ledit milieu de culture ou depuis ladite cellule.

11. Procédé selon la revendication 10, ledit produit de culture cellulaire étant choisi dans le groupe constitué par une protéine thérapeutique, une protéine de diagnostic, un polysaccharide, tel que l'héparine, un anticorps, un anticorps monoclonal, un facteur de croissance, une interleukine, un virus, une particule de type virus et une enzyme.
